**Europäisches Patentamt**

(19) **European Patent Office**

**Office européen des brevets**

(11) Veröffentlichungsnummer : **0 203 291**
**B1**

(12) **EUROPÄISCHE PATENTSCHRIFT**

(45) Veröffentlichungstag der Patentschrift :
07.06.89

(51) Int. Cl.⁴ : **A 61 B 6/14**, A 61 B 6/00

(21) Anmeldenummer : **86103606.9**

(22) Anmeldetag : **18.03.86**

(54) **Zahnmedizinische Röntgeneinrichtung.**

(30) Priorität : **01.04.85 DE 3511876**

(43) Veröffentlichungstag der Anmeldung :
**03.12.86 Patentblatt 86/49**

(45) Bekanntmachung des Hinweises auf die Patenterteilung : **07.06.89 Patentblatt 89/23**

(84) Benannte Vertragsstaaten :
**DE FR GB IT**

(56) Entgegenhaltungen :
**GB—A— 746 599**
**SE—A— 201 217**
**US—A— 2 369 453**
**US—A— 3 575 368**
**US—A— 3 617 742**

(73) Patentinhaber : **Siemens Aktiengesellschaft Berlin**
**und München**
**Wittelsbacherplatz 2**
**D-8000 München 2 (DE)**

(72) Erfinder : **Günther, Werner**
**Odenwaldstrasse 20**
**D-6140 Bensheim (DE)**
Erfinder : **Heubeck, Erich**
**Blütenweg 11**
**D-6140 Bensheim (DE)**

## Beschreibung

Insbesondere bei Röntgendiagnostikeinrichtungen, die im zahnmedizinischen Bereich zur Erstellung von Panorama-Röntgenaufnahmen des Kiefers eines Patienten eingesetzt werden, besteht das Problem, die aus Röntgengenerator und Röntgenfilmhalter bestehende Dreheinheit in einem bestimmten Bereich stufenlos in der Höhe verstellen zu können, um so die Einheit unterschiedlichen Patientengrößen (Kind/Erwachsener) oder unterschiedlichen Aufnahmetechniken (am sitzenden oder stehenden Patienten) anpassen zu können.

Bei bekannten solchen Einrichtungen (Prospekt M-D 80/1361 ORTHOPANTOMOGRAPH 10 ; WS 08832) ist die Dreheinheit auf einem Schlitten montiert, der an einem ortsfesten Ständer verstellbar gehaltert ist. Aus Stabilitätsgründen besteht der Ständer aus einteiligen, sich praktisch vom Fußboden bis zur Decke erstreckenden, im Querschnitt rechteckigen Rahmenteilen, an deren schmalen Seiten der Laufschlitten geführt ist. Die Rahmenteile bilden einen Hohlraum, in dem über einen Seilzug od. dgl. Gewichte aus Stahl oder Blei als Gewichtsausgleich für die Dreheinheit angeordnet sind.

Der praktisch raumhohe Ständer stellt nicht nur wegen seiner Länge und damit Sperrigkeit, sondern auch wegen seines Gewichts ein Verpackungs-, Transport- und Handling-Problem dar. Das mit etwa 90 kg anzusetzende Gegengewicht erschwert außerdem das Aufstellen und Montieren der Einrichtung. Wegen des relativ hohen Gewichts ist außerdem eine äußerst stabile und spielfreie Führung, die mit einem entsprechenden Kostenaufwand verbunden ist, erforderlich.

Aus der US-A-23 69 453 ist eine fahrbare Röntgen-Durchleuchtungseinrichtung bekannt, bei der auf einem mittels Rollen fahrbaren Unterteil ein aus zwei vertikal und parallel zueinander angeordneten Rohren bestehender Träger schwenkbar gelagert ist. Am Träger ist ein Laufwagen höhenverstellbar angeordnet, an dem ein Tragarm für einen Leuchtschirm und einen Röntgenstrahler befestigt ist. Die Rohre bestehen aus Montage- und Verpackungsgründen aus jeweils drei Abschnitten, einem oberen, mittleren und unterem Abschnitt. Am einen Rohr ist eine Zahnstange befestigt, die Teil der Höhenverstelleinrichtung für den Laufwagen ist.

Die Rohrabschnitte sind mittels einer Zapfen-Hülsen-Steckverbindung und mittels eines quer angeordneten Stifts, der in einer korrespondierend angeordneten Nut eingreift, miteinander verdrehsicher verbunden und werden mittels einer Zugstange axial miteinander verspannt.

Wenngleich sich durch die Querteilung der Rohre ein günstigerer Transport und eine einfachere Verpackung erzielen läßt, so ergibt sich durch das nach dem Zusammenstecken der Rohre notwendige Verspannen der gesamten Rohrkonstruktion mittels der Zugstange immer noch eine vergleichsweise umständliche und zeitaufwendige Montage. Ferner ist die außen an der Rohrwandung befestigte Zahnstange als Teil der Höhenverstelleinrichtung für den Laufwagen nicht nur optisch störend, sondern aus technischer Sicht ungünstig, weil die hierzu notwendigen Teile für die Höhenverstelleinrichtung sich einerseits relativ aufwendig gestalten und andererseits eine erhöhte Verschmutzungsgefahr darstellen.

Der im Patentanspruch angegebenen Erfindung liegt die Aufgabe zugrunde, demgegenüber eine verbesserte und vereinfachte Einrichtung anzugeben, mit der sich bezüglich des Aufbaus des Ständers, einschließlich der Führungsteile für den Laufschlitten, die angeführten Nachteile vermeiden und darüber hinaus die Herstellungs- und Montagekosten verringern lassen.

Vorteilhafte Ausgestaltungen und Weiterbildungen der Erfindung sind in den abhängigen Patentansprüchen enthalten.

Die Längenteilung der Standrohre liegt vorteilhafterweise in einem Bereich zwischen 1/3 und 2/3 der Gesamtlänge, wodurch sich einerseits eine akzeptable Verpackungsgröße und andererseits Vorteile in der Fertigung und Montage erzielen lassen.

Ein Ausführungsbeispiel der Erfindung wird nachfolgend anhand der Zeichnung näher beschrieben.

Anhand der Zeichnung wird ein Ausführungsbeispiel der Erfindung näher erläütert :

Es zeigen :

Figur 1 die erfindungsgemäße Einrichtung in einer schaubildlichen Darstellung,

Figuren 2 und 3 Alternativlösungen für einen Teil der Einrichtung nach Figur 1,

Figur 4 die Rohrrahmenkonstruktion nach Figur 1 alleine,

Figur 5 eine Prinzipskizze für die Verstelleinrichtung des Laufschlittens,

Figur 6 Einzelheiten betreffend Aufbau und Montage der Rohrkonstruktion und einer Fallsicherung für den Laufschlitten,

Figur 7 eine Alternativlösung zur Führung des Laufschlittens.

Figur 8 einen Schnitt entlang der Linie VIII/VIII in Figur 5.

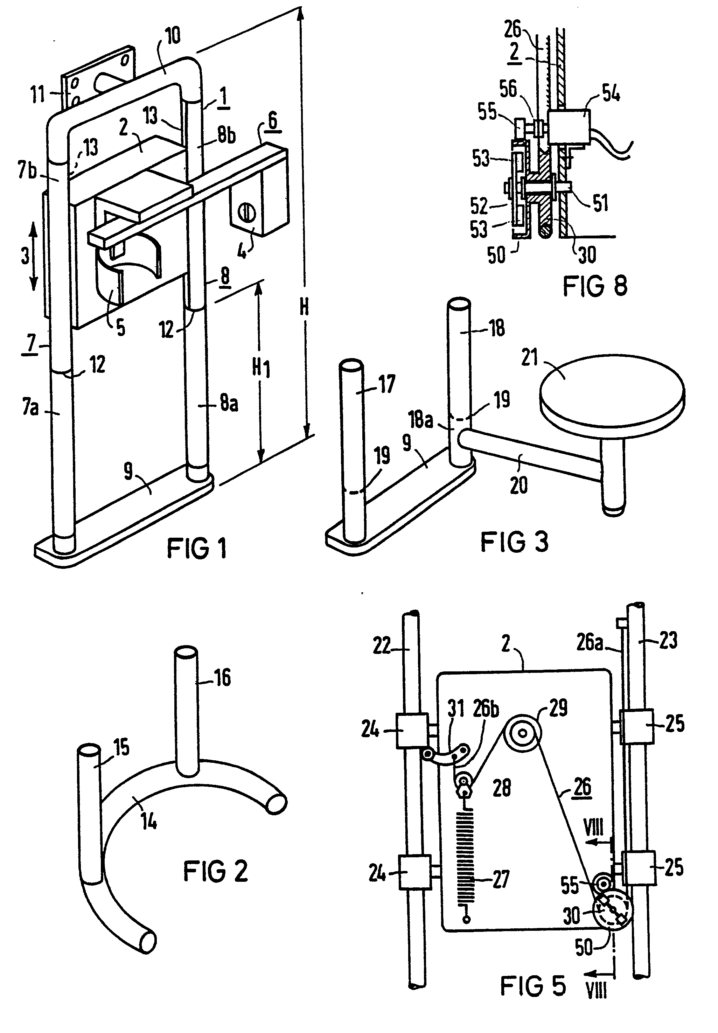

Die Figur 1 zeigt in einer schaubildlichen Darstellung eine zahnärztliche Röntgendiagnostikeinrichtung zur Erstellung von Panorama-Röntgenaufnahmen. Die Einrichtung besteht aus einem Ständer oder Stativ 1, an dem ein Laufschlitten 2 in Richtung des Pfeils 3 verstellbar gehalten ist. Am Laufschlitten 2 ist eine aus Röntgengenerator 4 und Röntgenfilmhalter 5 bestehende Dreheinheit 6 in üblicher Weise gehalten. Nachdem die Halterung der Dreheinheit für die Erfindung unbedeutend ist, wird diese nicht näher erläutert.

Der Ständer 1 besteht aus einem Rohrrahmen mit zwei parallelen vertikalen Tragrohren 7, 8, die an ihren Enden durch Querstreben 9 und 10 miteinander verbunden sind. Die Querstreben 9, 10 sind U-förmig ausgebildet; die deckenseitige Querstrebe 10 ist mit einem an einer Stellwand befestigbaren Abstandsglied 11 versehen.

Die Tragrohre 7, 8 sind an der mit 12 bezeichneten Stelle quergeteilt und lassen sich so in zwei Rohrabschnitte unterteilen, in einen unteren Rohrabschnitt mit den Rohren 7a, 8a und in einen oberen Rohrabschnitt mit den Rohren 7b, 8b. Die Rohre des unteren Rohrabschnittes weisen einen geschlossenen (kreisförmigen) Querschnitt auf, hingegen die Rohre 7b, 8b des oberen Rohrabschnittes mit einem Längsschlitz 13 versehen sind, aus dem Teile einer später noch näher erläuterten Führungseinrichtung zur Führung des Laufschlittens 2 vorstehen. Die Querteilung 12 erfolgt an einer Stelle, die etwa der untersten verlangten Höheneinstellung der Dreheinheit 6 entspricht; bei der heute üblichen Einstelltechnik beträgt diese Höhe ($H_1$), gerechnet vom Fußboden an, etwa 1 m bis 1,5 m bezogen auf eine Gesamtraumhöhe (H) von etwa 2,40 m.

Durch die Querteilung der Tragrohre ist es leicht möglich, anstelle der gezeigten, an einer Stellwand montierbaren Ständereinheit eine freistehende Ständereinheit vorzusehen oder im unteren Rohrabschnitt Zusatzgeräte, wie z. B. einen Patientenstuhl, zu integrieren. In den Figuren 2 und 3 sind zwei solcher Möglichkeiten aufgezeigt. Bei der einen Variante wird das in Figur 2 gezeigte Fußteil mit dem C-förmig gebogenen Standfuß 14 und den beiden Rohren 15 und 16 anstelle des unteren Rohrabschnittes mit den Rohren 7a, 8a und der Querstrebe 9 (Figur 1) gesetzt. Bei der Alternativlösung gemäß Figur 3 sind die beiden Standrohre 17, 18 an der mit 19 bezeichneten Stelle nochmals quergeteilt; das untere Rohrteil 18a des Standrohres 18 ist mit einem Ausleger 20 versehen, an dem ein Patientensitz 21 befestigt ist. Das Rohrteil 18a ist an der Querstrebe 9 drehbar gelagert, so daß der Sitz 21 um die Lagerachse des Rohrteils 18a geschwenkt werden kann.

Aufgrund des rohrförmigen Aufbaus des Ständers, welcher nicht auf die Verwendung zylindrischer Rohre, wie dargestellt, beschränkt ist, ist es möglich, unter Verwendung weitgehend gleicher Bauteile dem Kunden eine Vielfalt an verschiedenen Geräteausführungen anzubieten. Durch die sinnvolle Querteilung der vertikalen Tragrohre ergeben sich gegenüber den bisher bekannten Konstruktionen erheblich kleinere Verpackungsgrößen. Weitere Vorteile ergeben sich aus der nachfolgenden näheren Beschreibung der Konstruktion.

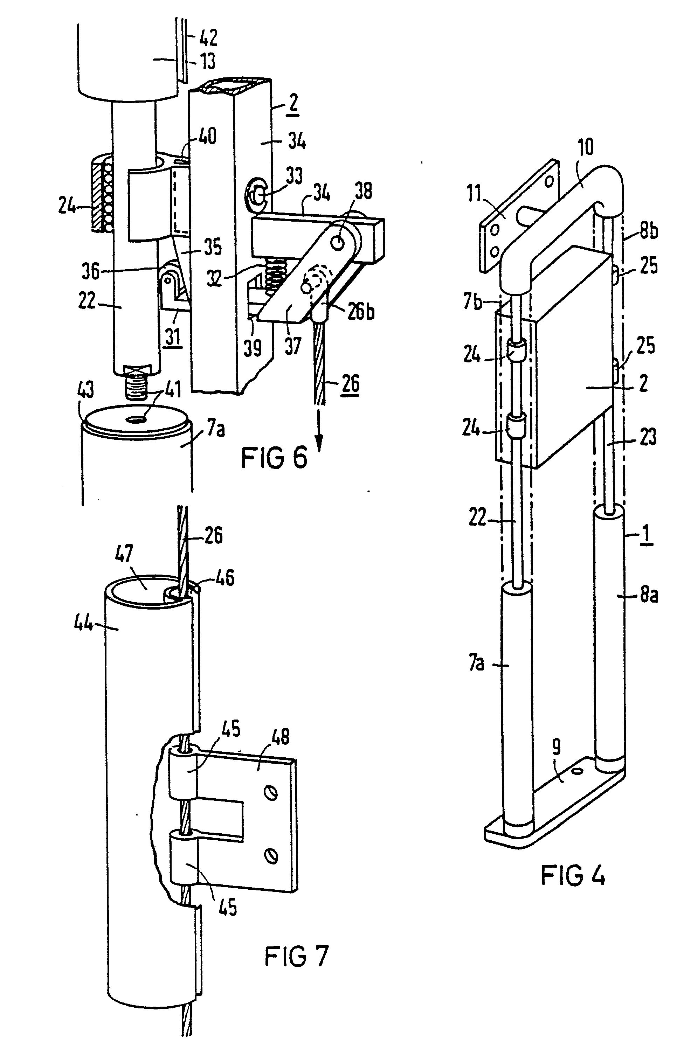

Die Figur 4 zeigt Einzelheiten des Ständeraufbaus. Die Rohre 7b, 8b des oberen Rohrabschnittes umschließen Führungsstangen 22, 23, auf denen Buchsen 24, 25 entlanggleiten. Die Buchsen 24, 25 sind mit dem Laufschlitten 2 verbunden, wobei die Buchsen 25 fest und die Buchsen 24 mit Spiel mit dem Laufschlitten 2 verbunden sind. Näheres ist aus den nachfolgenden Figuren ersichtlich. Die Führungsstangen 22, 23 sind einerseits mit den Schenkeln der oberen Querstrebe 10 und andererseits mit den Rohren 7a, 8a verbunden; zweckmäßigerweise kann diese Verbindung aus einer Schraubverbindung bestehen, wie diese beispielsweise in Figur 6 gezeigt ist.

Um das Gewicht der am Laufschlitten 2 befestigten Dreheinheit 6 kompensieren zu können, ist im Laufschlitten eine an sich bekannte Gewichtsausgleichsvorrichtung vorgesehen, welche in Figur 5 vom Prinzip her aufgezeigt ist. Die Ausgleichsvorrichtung enthält ein Zugseil 26, welches über eine beweglich geführte und mittels Zugfeder 27 belastete Umlenkrolle 28, eine Seilscheibe 29 und eine am Laufschlitten 2 ortsfest gelagerte weitere Umlenkrolle 30 geführt ist. Das eine Ende 26a des Zugseils 26 ist an der Führungsstange 23, das andere Ende 26b an einer Klemmeinrichtung 31 befestigt. Die sich bei Verstellung des Schlittens ändernde Federkraft wird durch die Seilscheibe 29 kompensiert, so daß das Gewicht des Laufschlittens und das der an ihm gehalterten Dreheinheit 6 in jeder Höhenlage ausgeglichen ist.

Die Klemmeinrichtung 31 ist Teil einer Fallsicherung, die anhand der Figur 6 näher erläutert wird.

Wie bereits dargelegt, ist der Laufschlitten 2 mittels der Führungsbuchsen 24, 25 in den Führungsstangen 22, 23 längsverschiebbar geführt. In Figur 6 ist hierzu der linke Teil der Führung näher dargestellt. Als Führungsbuchse 24 ist eine Kugelbuchse vorgesehen, an der ein Bolzen 33 befestigt ist, der mit dem Rahmen 34 des Laufschlittens 2 fest verbunden ist. Die Verbindung erfolgt auf der anderen Seite mit Spiel, d. h. der Bolzen und damit die Kugelbuchse 25 kann sich dort in Axialrichtung des Bolzens geringfügig zum Rahmen 34 hin bewegen, wodurch ein Verkanten des Laufschlittens in der Führung vermieden wird.

Die Führungsbuchse 24 enthält einen mit einer schrägen Fläche versehenen Keil 35, mit dem eine Klemmrolle 36 zusammenwirkt. Die Klemmrolle 36 ist an einem Kipphebel 37 gelagert, der wiederum mittels Lager 38 am Rahmen 34 kippbar gelagert ist. Das eine Ende 26b des Zugseils 26 ist am Kipphebel 37 eingehängt. Bei dem normalerweise vorhandenen Zug am Seil 26 wird der Kipphebel 37 gegen einen Anschlag 39 am Rahmen 34 gedrückt; in dieser Stellung hat die Rolle 36 keinen Kontakt mit der Führungsstange 22. Für den Fall, daß der Seilzug nicht gewährleistet ist, wird bei einer dadurch verbundenen Abwärtsbewegung des gesamten Laufschlittens die Klemmrolle 36 zwischen der Schräge des Keiles 35 und der Führungsstange 22 eingeklemmt, wodurch der Laufschlitten sofort arretiert wird. Eine zwischen Rahmen 34 und Kipphebel 37 angeordnete Zugfeder 32 unterstützt den Einklemmvorgang bei einem eventuell auftretenden Seilriß.

In Betrachtung der Figur 5 ist ersichtlich, daß das Seil 26 von der Umlenkrolle 30 aus über die beiden Führungsbuchsen 25 geführt ist. Hierzu sind entsprechende Freiräume in Form von z. B. Ausnehmungen, Durchbrüchen oder Schlitzen, wie in Figur 6 mit der Position 40 bezeichnet, vorhanden.

In Figur 6 ist weiterhin die Verbindung der Führungsstangen 22 (bzw. 23) mit den Tragrohren 7a (bzw. 8a) der unteren Rohrabschnitte dargestellt. Anstelle der gezeigten, aus Gewindezapfen und Gewindebohrung bestehenden Schraubverbindung (Position 41) kann auch eine andere adäquate Verbindung vorgesehen werden.

Im Bereich der Rohrabschnitte 7b, 8b wird die Führungseinrichtung von einer äußeren, den Längsschlitz 13 enthaltenden Hülle 42 umschlossen. Diese, in Figur 6 oben links dargestellte Hülle wird zentriert durch einen kleinen Ansatz 43 (links unten in Figur 6) im korrespondierenden Tragrohr.

Die Hülle 42 kann aus einem gerollten Blech gefertigt sein ; sie kann aber gemäß einer vorteilhaften Ausgestaltung der Erfindung so ausgebildet sein, daß sie gleichsam die Lagerung für die Führung des Laufschlittens bildet. Eine solche Alternativlösung ist in Figur 7 dargestellt. Hülle und Standrohr bestehen hier aus einem entsprechend dem dargestellten Profil gerollten Teil 44. Das Profil ist so gestaltet, daß ein den Führungsteilen 45 entsprechender zylindrischer Führungskanal 46 gebildet wird. Der verbleibende Hohlraum 47 kann ausgeschäumt werden, wodurch der Führungskanal 46 eine zusätzliche Steifigkeit bekommt. Der Laufschlitten ist bei dieser Alternativlösung an einem mit den Führungsteilen 45 verbundenen Trägerteil 48 befestigt. Zur Führung des Zugseils sind die Führungsteile 45 vorteilhafterweise als Buchsen ausgebildet.

Wie aus Figur 8, die einen Schnitt entlang der Linie VIII/VIII in Figur 5 zeigt, ersichtlich, ist die Umlenkrolle 30 mit einer topfartig ausgebildeten Trommel 50 aus weichmagnetischem Material verbunden. Trommel 50 und Umlenkrolle 30 sind auf einer am Laufschlitten 2 befestigten Achse 51 drehbar gehaltert. Auf der Achse 51 ist ein Hebel 52 befestigt, an dem zwei Elektromagnetspulen 53 angeordnet sind, welche mit der Trommel 50 einer Elektromagnetbremse zum Festhalten des Laufschlittens 2 — und damit der von ihm getragenen Dreheinheit 6 — in einer beliebig einstellbaren Höhenlage dient. Das Ein- und Abschalten der beiden Topfmagnete 53 kann in bekannter Weise durch einen in einem nicht dargestellten Stromkreis angeordneten Schalter oder Taster erfolgen.

Die Trommel 50 dient gleichsam auch zum motorischen Verstellen des Laufschlittens 2. Hierzu liegt am äußeren Trommelrand ein von einem Elektromotor 54 angetriebenes Reibrad 55 an. Eine zwischen Antriebsmotor 54 und Reibrad angeordnete Rutschkupplung 56 erlaubt auch eine Verstellung des Laufschlittens von Hand.

## Patentansprüche

1. Medizinische, insbesondere zahnmedizinische, Einrichtung mit einem ortsfest aufstellbaren Ständer (1), an dem mittels einer Führungseinrichtung (22, 23 ; 24, 25) ein Laufschlitten (2) verstellbar gehaltert ist, der Träger eines medizinischen Gerätes, z. B. einer aus Röntgengenerator (4) und Röntgenfilmhalter (5) bestehenden Dreheinheit (6), ist, wobei der Ständer (1) aus einem Rohrrahmen gebildet ist, welcher zwei parallel verlaufende und dazwischen den Laufschlitten (2) aufnehmende, senkrechte Tragrohre (7, 8) enthält, die zumindest an ihren einen Enden durch eine Querverstrebung (9, 10) miteinander verbunden sind, und die innerhalb der Rohrlänge zumindest eine Querteilung (12) aufweisen, an der die geteilten Rohrabschnitte (7a, 8b ; 8a, 8b) bei Montage drehfest miteinander verbunden werden, dadurch gekennzeichnet, daß die beiden Tragrohre (7, 8) aus einem bodenseitigen, bis zur Querteilung (12) sich erstreckenden unteren Rohrabschnitt (7a, 8a) mit Rohren mit im Querschnitt geschlossenem Profil und aus einem sich daran anschließenden und mit dem unteren Rohrabschnitt (7a, 8a) verbundenen oberen Rohrabschnitt (7b, 8b) mit Rohren (42) mit im Querschnitt einseitig offenem und dadurch einen Längsschlitz (13) bildenden Profil bestehen und daß der obere Rohrabschnitt (7b, 8b) Führungsglieder (22, 23, 46) für eine Verstellung des zwischen den Tragrohren (7, 8) dieses Abschnittes angeordneten Laufschlittens (2) enthält.

2. Einrichtung nach Anspruch 1, dadurch gekennzeichnet, daß die Rohre des mit Längsschlitzen (13) versehenen Rohrabschnites (7b, 8b) jeweils eine, vorzugsweise zentrisch angeordnete, mit den boden- und deckseitigen Rahmenelementen (7a, 8a ; 10) verbindbare Führungsstange (22, 23) enthalten, auf der mit dem Laufschlitten (2) verbundene Führungsbuchsen (24, 25) entlanggleiten, und daß die Führungsstangen (22, 23) von einem den Längsschlitz (13) enthaltenden und im Außendurchmesser den sich daran anschließenden Teilen (7a, 8a ; 10) entsprechenden Hüllrohr (42) umgeben ist.

3. Einrichtung nach Anspruch 1, dadurch gekennzeichnet, daß die Rohre des mit Längsschlitzen versehenen Rohrabschnittes (7b, 8b) durch ein Hüllrohr (44) gebildet werden, welches so gestaltet ist, daß es einen Führungskanal (46) bildet, in dem ein Führungsteil (45), an dem der Laufschlitten (2) befestigt ist, entlanggleiten kann.

4. Einrichtung nach Anspruch 2 oder 3, dadurch gekennzeichnet, daß das Hüllrohr (44) aus einem gerollten Blech besteht.

5. Einrichtung nach Anspruch 1, dadurch gekennzeichnet, daß die Führungselemente (23, 24, 25) zur Führung des Laufschlittens (2) an der einen Seite des Laufschlittens fest und an der anderen Seite mit Spiel mit dem Laufschlitten (2) verbunden sind.

6. Einrichtung nach Anspruch 1, dadurch gekennzeichnet, daß im Laufschlitten (2) eine Federgewichtsausgleichsvorrichtung (26 bis 30) vorgesehen ist mit einer Zug- oder Druckfeder (27) und einem über Umlenkrollen (28, 30) und einer Kurven(seil)scheibe (29) geführten Zugseil (26), dessen eines Ende (26a) an einer der Führungsstangen (23) im Rohrrahmen (34) und dessen anderes Ende (26b) am Laufschlitten (2) befestigt ist.

7. Einrichtung nach Anspruch 6, dadurch gekennzeichnet, daß der Laufschlitten (2) mit einer Fallsicherungsvorrichtung (35 bis 38) versehen ist, die aus einem zwischen Führungsrohr (22, 23) und einer mit dem Laufschlitten (2) verbundenen schrägen Fläche (35) einklemmbaren, mit dem einen Ende (26b) des Zugseils (26) verbundenen Klemmkörper (36) besteht, der infolge der Federwirkung der Zug- oder Druckfeder (26) bei nicht vorhandenem Seilzug in Klemmeingriffsposition und bei vorhandenem Seilzug in Außereingriffsposition kommt.

8. Einrichtung nach Anspruch 1, dadurch gekennzeichnet, daß die beiden Tragrohre (7, 8) durch U-förmig gestaltete Querverstrebungen (9, 10) miteinander verbunden sind und an ihren den Tragrohrabstand bestimmenden Enden Verbindungselemente in Form von z. B. Steck- oder Schraubbuchsen und -zapfen (41) für eine leichte Verbindung mit dem Tragrohren (7, 8) enthalten.

9. Einrichtung nach Anspruch 8, dadurch gekennzeichnet, daß an dem mit der bodenseitigen Querverbindung (9) verbundenen Rohrabschnitt (18) ein schwenkbarer Ausleger (20) mit einem Patientensitz (21) gehaltert ist.

10. Einrichtung nach Anspruch 9, dadurch gekennzeichnet, daß der Ausleger (20) an einem durch eine weitere Querteilung (19) gebildeten Rohrelement (18a) befestigt ist und die weitere Querteilung (19) unter Bildung gleicher Rohrlängen auch am gegenüberliegenden Rohr (17) dieses Rohrabschnittes vorhanden ist.

11. Einrichtung nach Anspruch 8, dadurch gekennzeichnet, daß die bodenseitige Querverstrebung (9) einen die Einrichtung frei tragenden, vorzugsweise C-förmig ausgebildeten, Standfuß (14) bildet.

12. Einrichtung nach Anspruch 11, dadurch gekennzeichnet, daß von dem mit der bodenseitigen Querversteifung (9) verbundenen Rohrabschnitt (7a, 8a) das eine Rohr (8a) am Standfuß (9) fest und das andere (7a) in der Höhe einstellbar befestigt ist.

13. Einrichtung nach Anspruch 8, dadurch gekennzeichnet, daß an der oberen Querverstrebung (10) ein einen Abstand zu einer Stellwand festlegendes Abstützelement (11) vorgesehen ist.

## Claims

1. A medical, in particular dental, apparatus having a stationary, adjustable stand (1), on which there is adjustably mounted, by means of a guiding device (22, 23 ; 24, 25), a carriage (2) which is the support for a medical device, e. g. a rotary unit comprising an X-ray generator (6) and an X-ray film hoder (5), wherein the stand (1) comprises a tubular frame which includes two parallel vertical supporting tubes (7, 8), accommodating the carriage (2) therebetween, which are connected to one another at least at their one end by a cross strut (9, 10) and which have within the length of the tube at least one transverse division at which the divided tubular sections (7a, 8b ; 8a, 8b) are connected to one another so as not to rotate when assembled, characterised in that the two supporting tubes (7, 8) comprise a lower tube section (7a, 8a) at the bottom extending up to the tranverse division (12) and having tubes with closed cross-sectional profiles, and an upper tubular section (7b, 8b) next to and connected to the lower tube section (7a, 8a) and having tubes (42) with a cross-section open at one side and thereby forming a longitudinal slit (13), and in that the upper tube section (7b, 8b) includes guiding members (22, 23, 46) for adjusting the carriage (2) arranged between the supporting tubes (7, 8) of this section.

2. An apparatus according to claim 1, characterised in that the tubes of the tube section (7b, 8b) provided with longitudinal slits (13) each have a guide rod (22, 23), preferably arranged centrally, which can be connected to the bottom and top frame elements (7a, 8a ; 10) and along which guide sleeves (24, 25) connected to the carriage (2) slide, and that the guide rods (22, 23) are surrounded by a tubular jacket (42) including the longitudinal slit (13) and with its outer diameter corresponding to the parts (7a, 8a ; 10) connected thereto.

3. An apparatus according to claim 1, characterised in that the tubes of the tube section (7b, 8b), provided with longitudinal slits are formed by a jacket tube (44) which is shaped so that it forms a guiding passage (46) in which a guiding part (45), to which the carriage (2) is attached, can slide along.

4. An apparatus according to claim 2 or claim 3, characterised in that the jacket tube (44) consists of a rolled sheet.

5. An apparatus according to claim 1, characterised in that the guiding elements (23, 24, 25) for guiding the carriage (2) are securely connected to one side of the carriage and with play to the other side of the carriage (2).

6. An apparatus according to claim 1, characterised in that provided in the carriage (2) is a spring counter-balance device (26 to 30) having a tension or pressure spring (27) and a traction line (26) guided over guide rollers (28, 30) and a cam (line) disc (29) whose one end (26a) is attached to one of the guide rods (23) in the tubular frame (34) and whose other end (26b) is attached to the carriage (2).

7. An apparatus according to claim 6, characterised in that the carriage (2) is provided with a falling safety device (35 to 38) which consists of a clamping part (36), connected to one end (26b) of the traction line (26), which can be clamped in between, the guide tube (22, 23) and an inclined surface (35) connected to the carriage (2), and which, as a result of the spring action of the tension or pressure spring (26), comes into a clamping engagement position when there is no tension on the line, and a disengaged position when there is tension on the line.

8. An apparatus according to claim 1, characterised in that the two supporting tubes (7, 8) are connected to one another by means of U-shaped cross struts (9, 10) and comprise, at their ends that determine the distance between the supporting tubes, connecting elements in the form of e. g. push-in or screw sockets and plugs (41) for easy connection to the supporting tubes (7, 8).

9. An apparatus according to claim 8, characterised in that mounted on the tube section (18) connected to the bottom cross connection (9) is a pivotable arm with a patient seat (21).

10. An apparatus according to claim 9, characterised in that the arm (20) is attached to a tube element (18a) formed by a further transverse division (19) and the further transverse division (19) is also present and forms the same tube lengths in the opposite tube (17) of this tube section.

11. An apparatus according to claim 8, characterised in that the bottom cross strut (9) forms a base (14), preferably C-shaped, freely carrying the apparatus.

12. An apparatus according to claim 11, characterised in that of the tube section (7a, 8a), connected to the bottom cross reinforcement (9), one tube (8a) is securely fixed to the base (9) and the other tube (7a) is attached so that its height can be adjusted.

13. An apparatus according to claim 8, characterised in that a supporting element (11) determining a distance from a movable partition is provided on the upper cross strut (10).

**Revendications**

1. Dispositif médical, notamment dentaire, comportant un support (1), qui peut être installé de façon fixe et sur lequel est monté de manière à s'y déplacer, au moyen d'un dispositif de guidage (22, 23 ; 24, 25), un chariot (2), qui supporte un appareil médical, par exemple une unité rotative (6) constituée par un générateur radiologique (4) et un support (5) de film radiographique, et dans lequel le support (1) est formé par un cadre tubulaire comprenant deux tubes verticaux parallèles de support (7, 8), qui reçoivent entre eux le chariot (2) et sont reliés entre eux, au moins au niveau de premières extrémités, par une traverse (9, 10) et possèdent, dans l'intervalle s'étendant sur la longueur du tube, une subdivision transversale (12), au niveau de laquelle les sections séparées (7a, 7b ; 8a, 8b) des tubes sont reliées entre elles avec blocage en rotation lors du montage, caractérisé par le fait que les deux tubes de support (7, 8) sont constitués par une section inférieure (7a, 8a), qui est située du côté du sol, s'étend jusqu'à la division transversale (12) et comporte des tubes possédant un profil à section transversale fermée, et par une section supérieure (7b, 8b), qui se raccorde et est reliée à la section inférieure (7a, 8a) et comporte des tubes (42) possédant un profil s'ouvrant d'un côté en coupe transversale et formant de ce fait une fente longitudinale (13), et que la section supérieure (7b, 8b) contient des organes de guidage (22, 23, 46) permettant le déplacement du chariot (2) disposé entre les tubes de support (7, 8) de cette section.

2. Dispositif suivant la revendication 1, caractérisé par le fait que les tubes de la section (7b, 8b) de tube possédant des fentes longitudinales (13) contiennent des barres respectives de guidage (22, 23), qui sont disposées de préférence d'une manière centrée et peuvent être reliées aux éléments (7a, 8a ; 10) situés du côté du sol et à la partie supérieure, et sur lesquelles glissent des manchons de guidage (24, 25) reliés au chariot (2), et que les barres de guidage (22, 23) sont entourées par un tube enveloppe (42) contenant la fente longitudinale (13) et dont le diamètre extérieur correspond à celui des parties (7a, 8a ; 10) qui s'y raccordent.

3. Dispositif suivant la revendication 1, caractérisé par le fait que les tubes de la section (7b, 8b) munie de fentes longitudinales sont formés par un tube enveloppe (44) agencé de manière à former un conduit de guidage (46), dans lequel peut glisser un élément de guidage (45), sur laquelle est fixé le chariot (2).

4. Dispositif suivant la revendication 2 ou 3, caractérisé par le fait que le tube enveloppe (44) est constitué par une tôle laminée.

5. Dispositif suivant la revendication 1, caractérisé par le fait que les éléments de guidage (23, 24, 25) servant à guider le chariot (2) sont reliés rigidement à un côté du chariot et son reliés avec jeu au chariot (2), sur l'autre côté.

6. Dispositif suivant la revendication 1, caractérisé par le fait que dans le chariot (2) il est prévu un dispositif de contrepoids à ressort (26 à 30) comportant un ressort de traction ou de pression (27) et un câble de traction (26), guidé sur des galets de renvoi (28, 30) et une poulie de guidage (à câble) (29) et dont une extrémité (26a) est fixée à l'une des barres de guidage (23) dans le cadre tubulaire (34), et dont l'autre extrémité (26b) est fixée au chariot (2).

7. Dispositif suivant la revendication 6, caractérisé par le fait que le chariot (2) comporte un dispositif de sécurité anti-chute (35 à 38), constitué par un corps de serrage (36), qui peut être serré entre le tube de guidage (22, 23) et une surface oblique (35) reliée au chariot (2), est relié à une extrémité (26b) du câble de traction (26) et, sous l'effet de l'action du ressort de traction ou de compression (26), vient dans une position d'engagement avec serrage lorsque le câble de traction est absent et dans une position dégagée lorsque le câble de traction est présent.

8. Dispositif suivant la revendication 1, caractérisé par le fait que les deux tubes de support (7, 8) sont reliés entre eux par des traverses en forme de U (9, 10) et contiennent, au niveau de leurs extrémités déterminant la distance entre les tubes de support, des éléments de liaison se présentant sous la forme de manchons et de broches (41) d'enfichage ou de vissage, permettant une liaison facile avec les tubes de support (7, 8).

9. Dispositif suivant la revendication 8, caractérisé par le fait qu'un bras en console pivotant (20) muni d'un siège (21) pour le patient est maintenu sur la section de tube (18) reliée à la liaison transversale (9) située du côté du sol.

10 Dispositif suivant la revendication 9, caractérisé par le fait que le bras en console (20) est fixé à un élément de tube (18a) délimité par une autre subdivision transversale (19), et l'autre subdivision transversale (19) est ménagée de manière à former des longueurs de tubes identiques également sur le tube opposé (17) de cette section de tubes.

11. Dispositif suivant la revendication 8, caractérisé par le fait que la traverse (9), située du côté du sol, forme un pied de support (14), réalisé de préférence avec la forme d'un C supportant le dispositif.

12. Dispositif suivant la revendication 11, caractérisé par le fait qu'un tube (8a) de la section de tubes (7a, 8a), reliée à la traverse (9) située du côté du sol, est fixé rigidement sur le pied de support (9) et que l'autre tube (7a) est fixé de manière à être réglable en hauteur.

13. Dispositif suivant la revendication 8, caractérisé par le fait qu'un élément du support (11) fixant une distance par rapport à une paroi de montage est prévu sur la traverse supérieure (10).

FIG 1

FIG 2

FIG 3

FIG 5

FIG 8

FIG 6

FIG 7

FIG 4

2